# EUROPÄISCHE PATENTSCHRIFT

(11) **EP 3 529 233 B1**
(45) Veröffentlichungstag und Bekanntmachung des Hinweises auf die Patenterteilung: **12.08.2020**
(21) Anmeldenummer: 17790722.7
(22) Anmeldetag: 16.10.2017
(51) Int. Cl.: C07C 319/14, C07C 323/09, C07C 323/20, C07C 323/62, C07D 257/06

(54) **VERFAHREN ZUR HERSTELLUNG VON 3-ALKYLSULFANYL-2-CHLOR-N-(1-ALKYL-1H-TETRAZOL-5-YL)-4-TRIFLUOROMETHYL-BENZAMIDEN**
METHOD FOR THE PREPARATION OF 3-ALKYLSULFANYL-2-CHLOR-N- 1-ALKYL-1H-TETRAZOL-5-YL) -4-TRIFLUOROMETHYL-BENZAMIDENE
PROCÉDÉ DE FABRICATION DE BENZAMIDES 3-ALKYLSULFANYL-2-CHLOR-N-(1-ALKYL-1H-TÉTRAZOL-5-YL)-4-TRIFLUOROMÉTHYL

(30) Priorität: 20.10.2016 EP 16194840
(43) Veröffentlichungstag der Anmeldung: 28.08.2019
(73) Patentinhaber: Bayer CropScience Aktiengesellschaft, 40789 Monheim am Rhein (DE)
(72) Erfinder: GALLENKAMP, Daniel, 42113 Wuppertal (DE); FORD, Mark, James, 65207 Wiesbaden-Breckenheim (DE)
(74) Vertreter: BIP Patents
(86) Internationale Anmeldenummer: PCT/EP2017/076307
(87) Internationale Veröffentlichungsnummer: WO 2018/073157

(56) Entgegenhaltungen:
- WO-A1-2012/028579
- WO-A2-2009/149806

## Beschreibung

Die Erfindung betrifft ein Verfahren zur Herstellung von 3-Alkylsulfanyl-2-chlor-N-(1-alkyl-1*H-*tetrazol-5-yl)-4-trifluoromethyl-benzamiden, die als agrochemisch wirksame Stoffe von Nutzen sind. Insbesondere betrifft die Erfindung ein Verfahren zur Herstellung von 2-Chlor-3-methylsulfanyl-N-(1-methyl-1*H*-tetrazol-5-yl)-4-trifluoromethyl-benzamid in seiner stabilen Kristallmodifikation.

Aus WO 2012/028579 A1 sind eine Vielzahl von agrochemisch wirksamen N-(Tetrazol-5-yl)-arylcarbonsäureamiden bekannt. Als besonders vorteilhaft haben sich 3-Alkylsulfanyl-2-chlor-N-(1-alkyl-1*H*-tetrazol-5-yl)-4-trifluoromethyl-benzamide erwiesen. Die zu deren Herstellung notwendigen 3-Alkylsulfanyl-2-chlor-4-trifluoromethyl-benzoesäuren können nach einem in WO2009/149806 A1 beschriebenen Verfahren hergestellt werden. Dieses Verfahren ist jedoch aufgrund der geringen Ausbeuten und teuren Ausgangsmaterialien nicht für eine technische Synthese in großem Maßstab anwendbar. Zudem führt die Herstellung nach einem in WO 2012/028579 A1 beschriebenen Verfahren im Falle der Verbindung 2-Chlor-3-(methylsulfanyl)-N-(1-methyl-1*H*-tetrazol-5-yl)-4-(trifluormethyl)benzamid nicht zu deren in WO 2017/005585 A1 beschriebenen stabilen Kristallmodifikation, die erhebliche anwendungstechnische Vorteile aufweist.

Aufgabe der vorliegenden Erfindung ist die Bereitstellung eines Verfahrens zur Herstellung von 3-Alkylsulfanyl-2-chlor-N-(1-alkly-1*H*-tetrazol-5-yl)-4-trifluoromethyl-benzamiden, welches die Nachteile der aus dem Stand der Technik bekannten Verfahren überwindet.

Es wurde nun gefunden, dass 3-Alkylsulfanyl-2-chlor-N-(1-alkyl-1*H*-tetrazol-5-yl)-4-trifluoromethyl-benzamide - ausgehend von 2,3-Dichlorbenzotrifluorid - hergestellt werden können durch die Reaktionsfolge einer Alkylthiolierung, Carboxylierung und anschließender Amidierung.

Ein Gegenstand vorliegender Erfindung ist somit ein Verfahren zur Herstellung von 3-Alkylsulfanyl- 2-chlor-N-(1-alkyl-1*H*-tetrazol-5-yl)-4-trifluoromethyl-benzamiden der allgemeinen Formel (I), dadurch gekennzeichnet, dass
a) in einem ersten Schritt 2,3-Dichlorbenzotrifluorid (II) mit einem Thiolat (IV) zu einem 2-Alkysulfanyl-3-chlor-benzotrifluorid (III) umgesetzt wird,
b) in einem zweiten Schritt das 2-Alkysulfanyl-3-chlor-benzotrifluorid (III) mit einem metallorganischen Reagens zu [2-Chlor-3-(alkylsulfanyl)-4-(trifluormethyl)phenyl] Metall Anion (V) und anschließend mit einem Carboxlierungsreagenz zu 3-Alkylsulfanyl-2-chlor-4-trifluormethylbenzoesäure (VI) umgesetzt wird, und
c) in einem dritten Schritt mit einem Aktivator in Anwesenheit einer Base und eines Acyltransfer Reagenz die Amidierung mit einem 5-Amino-1-alkyltetrazol (VII) zum 3-Alkylsulfanyl- 2-chlor-N-(1-alkyl-1*H*-tetrazol-5-yl)-4-trifluoromethyl-benzamid (I) erfolgt: 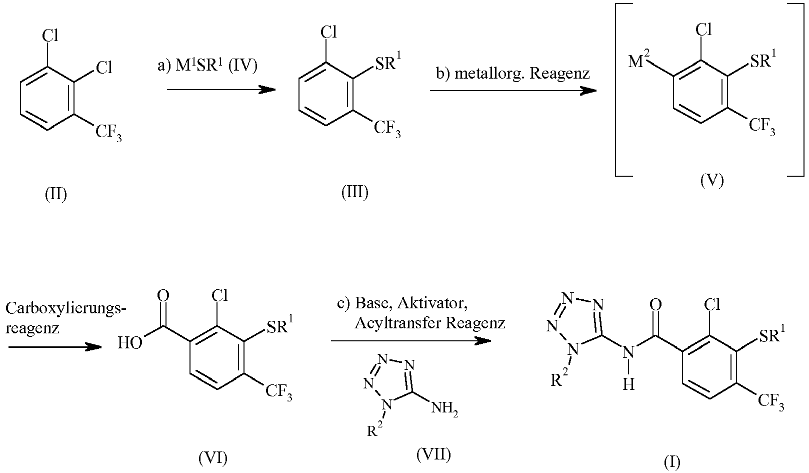 und
d) worin die Substituenten wie nachfolgend definiert sind:
   - R¹: bedeutet C₁-C₄-Alkyl oder durch s Reste aus der Gruppe bestehend aus Chlor, Fluor, Methoxy und Ethoxy substituiertes Phenyl,
   - R²: bedeutet C₁-C₄-Alkyl oder durch s Reste aus der Gruppe bestehend aus Chlor, Fluor, Methoxy und Ethoxy substituiertes Phenyl,
   - M¹: bedeutet Lithium, Natrium oder Kalium,
   - M²: bedeutet Li, ZnX, MgX,
   - X: bedeutet Chlor, Brom oder Iod,
   - s: bedeutet 0, 1, 2 oder 3.

Wesentliche Vorteile des erfindungsgemäßen Verfahrens sind:
- die Nutzung leicht erhältlicher Ausgangsmaterialien,
- die regioselektive Reaktion in Schritten a) und b),
- die direkte Amidierung der Benzoesäure ohne Isolierung eines intermediär gebildeten Benzoesäurechlorids,
- die Bildung der stabilen Kristallmodifikation mindestens für den Fall, daß R für Methyl steht und
- die hohe Gesamtausbeute.

In den Formeln (I), (III), (IV), (V), (VI) und (VII) können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl.

Vorzugsweise steht R¹ und R² für C₁-C₄-Alkyl, M¹ bedeutet Natrium und M² bedeutet Lithium. Besonders bevorzugt stehen R¹ und R² jeweils für Methyl.

Die hier verwendeten Ausgangsmaterialien sind entweder kommerziell erhältlich oder durch einfache dem Fachmann bekannte Methoden zugänglich.

### Erster Schritt des erfindungsgemäßen Verfahrens:

Die Verbindung (IV) wird in einem Mengenverhältnis von 1:1 bis 2:1 Moläquivalenten bezogen auf die Verbindung der allgemeinen Formel (II) eingesetzt. Bevorzugt ist ein Mengenverhältnis von 1:1 bis 1,5:1, besonders bevorzugt 1,3:1. Üblicherweise wird eine wässrige Lösung der Verbindung (IV) eingesetzt. Besonders gut geeignet sind Natriumthiomethylat (NaSMe) und Kaliumthiomethylat (KSMe).

Die Verbindungen der allgemeinen Formel (IV) können sowohl in-situ als auch ex-situ aus den entsprechenden Thiolen und einer Base, wie Carbonaten, Hydrogencarbonaten, Alkalimetallhydroxide, Erdalkalimetallhydroxide und organischen Basen, hergestellt werden. Geeignete Basen sind LiOH, NaOH, KOH, Ca(OH)₂, Na₂CO₃, K₂CO₃, Li₂CO₃, Cs₂CO₃, NaHCO₃, KHCO₃, NaOAc, KOAc, LiOAc, NaOMe, NaOEt, NaO-t-Bu, KO-t-Bu, Trialkylamine, Alkylpyridine, Phosphazene und 1,8-Diazabicyclo[5.4.0]undecen.

Die Reaktion wird in der Regel in einer wässrigen Lösung des Thiolats mit einem Phasentransferkatalysator ohne weiteres Lösungsmittel durchgeführt. Die Reaktion kann auch in einem Lösungsmittel durchgeführt werden. Geeignete Lösungsmittel sind Ether wie Diethylether, Diisopropylether, Methyl-t-butylether, THF, Methyl-THF, Dioxan, 1,2-Dimethoxyethan, Diglyme oder Anisol; aromatische Lösungsmittel wie Toluol, Xylol, Chlorbenzol oder 1,2-Dichlorbenzol; aliphatische Kohlenwasserstoffe wie n-Hexan, n-Heptan, Cyclohexan oder Methylcyclohexan. Bevorzugt sind Methyl-t-butylether, Toluol, Chlorbenzol, 1,2-Dichlorbenzol, n-Heptan oder Methylcyclohexan.

Als Phasentransferkatalysator werden Ammonium- oder Phosphonium-Salze, wie Methyltributylammoniumchlorid, Methyltributylammoniumbromid, Methyltrioctylammoniumchlorid, Methyltrioctylammoniumbromid, Tetrahexylammoniumchlorid, Tetrahexylammoniumbromid, Tetrahexylammoniumiodid, Tetraoctylammoniumchlorid, Tetraoctylammoniumbromid, Tetraoctylammoniumiodid, Tributylhexadecylammoniumchlorid, Tributylhexadecylammoniumbromid, Dimethyldidecylammoniumchlorid, Dimethyldodecylbenzylammoniumchlorid, Tetrabutylammoniumchlorid, Tetrabutylammoniumbromid, Tetrabutylammoniumhydrogensulfat, Benzyltributylammoniumchlorid, Benzyltributylammoniumbromid, Aliquat HTA-1®, Aliquat 134®, Tributyltetradecylphosphoniumchlorid, Tributyltetradecylphosphoniumbromid, Tributylhexadecylphosphoniumbromid, Tetraoctylphosphoniumbromid, Trihexyltetradecylphosphoniumchlorid, Trihexyltetradecylphosphoniumbromid. Bevorzugt sind Aliquat 134® und Tributyltetradecylphosphoniumchlorid. Der Phasentransferkatalysator wird in einem Mengenverhältnis von 0,1 bis 10 Molprozent bezogen auf die Verbindung der allgemeinen Formel (II) eingesetzt. Bevorzugt sind 1 bis 6 Molprozent, besonders bevorzugt 2 bis 4 Molprozent.

Die Reaktion wird in der Regel bei einer Temperatur von 20 bis 80° C, vorzugsweise 50 bis 80° C, besonders bevorzugt 70 bis 80° C, durchgeführt. Die vollständige Umsetzung ist in der Regel nach 5 bis 12 Stunden erfolgt. Die Reaktion kann auch unter erhöhtem oder veringertem Druck durchgeführt werden.

### Zweiter Schritt des erfindungsgemäßen Verfahrens:

Die Verbindung der Formel (III) wird wahlweise mit oder ohne katalytische Mengen eines Amins in einem inerten, aprotischen Lösungsmittel bei tiefer Temperatur vorgelegt. Dann wird eine Alkyllithium Verbindung als Metallierungsreagenz langsam zudosiert. Nach beendeter Dosierung kann durch Zugabe eines entsprechenden Metallsalzes bei tiefer Temperatur eine Ummetallierung der zunächst gebildeten [2-Chlor-3-(alkylsulfanyl)-4-(trifluormethyl)phenyl]lithium Verbindung erfolgen. Die Verbindungen der allgemeinen Formel (V) werden nicht isoliert sondern durch Zugabe eines Carboxylierungsreagenzes direkt weiter umgesetzt. Das Carboxylierungsreagenz wird dabei solange zudosiert bis keine exotherme Reaktion mehr erkennbar ist und die Verbindung der allgemeinen Formel (V) vollständig zur Verbindung der allgemeinen Formel (VI) umgesetzt ist.

Als Carboxylierungsreagenz können beispielsweise Kohlendioxid, Chlorameisensäureester oder Isocyanate verwendet werden. Bevorzugt ist Kohlendioxid.

Die Reaktion wird unter wasserfreien Bedingungen in einem inerten, aprotischen Lösungsmittel durchgeführt. Als inerte, aprotische Lösungsmittel geeignet sind C₅-C₈ lineare, verzweigte oder cyclische Kohlenwasserstoffe wie Pentan, Hexan, Cyclohexan, Heptan, Methylcyclohexan, Isooctan sowie Ether wie beispielsweise Tetrahydrofuran, 2-Methyltetrahydrofuran, Dioxan, Diethylether, tert-Butylmethylether, Cyclopentylmethylether und Glykolether. Ether als Lösungsmittel sind generell bevorzugt sowie Mischungen aus Kohlenwasserstoffen und Ethern. Besonders bevorzugt sind Mischungen aus Tetrahydrofuran und Kohlenwasserstoffen.

Als Metallierungsreagenz eignen sich Alkyllithium Verbindungen bzw. Lithiumamid Verbindungen wie Lithiumdiisopropylamid oder Lithium-2,2,6,6-tetramethylpiperidid, die als eine starke Base fungieren. Bevorzugt sind kommerziell erhältliche Alkyllithium Verbindungen wie Methyllithium, Ethyllithium, iso-Propyllithium, n-Butyllithium, iso-Butyllithium, sec-Butyllithium, n-Pentyllithium, Neopentyllithium, n-Hexyllithium und 2-(Ethylhexyl)lithium. Besonders bevorzugt ist n-Butyllithium. Die Alkyllithium Verbindung wird in einem Mengenverhältnis von 0,9:1 bis 1,2:1 bezogen auf die Verbindung der allgemeinen Formel (III) eingesetzt. Bevorzugt ist ein Verhältnis von 0,95:1 bis 1,1:1, besonders bevorzugt ein Verhältnis von 1:1.

Als Metallsalze können equimolare Mengen der entsprechenden Zink- oder Magnesiumhalogenide wie bespielsweise ZnCl₂, ZnBr₂, MgCl₂, MgBr₂ oder MgBr₂·OEt₂ verwendet werden. Vorteil einer Ummetallierung auf M² = ZnX, MgX ist die erhöhte Stabilität der entsprechenden [2-Chlor-3-(alkylsulfanyl)-4-(trifluormethyl)phenyl] Metall Verbindung bei 0 bis 23 °C im Vergleich zu M² = Li. Für M² = Zn ist die Reaktivität gegenüber Elektrophilen deutlich herabgesetzt, wodurch keine Reaktion mit Kohlendioxid erfolgt.

Durch die Verwendung katalytischer Mengen eines Amins kann die Ausbeute sowie die Reinheit der Verbindung der allgemeinen Formel (VI) im Vergleich zur alleinigen Verwendung einer Alkyllithium Verbindung gesteigert werden. Geeignete Amine sind primäre oder sekundäre Amine wie beispielsweise n-Propylamin, Diethylamin, Diisopropylamin oder 2,2,6,6-Tetramethylpiperidin. Bevorzugt wird Diisopropylamin verwendet. Das Amin wird in einem Mengenverhältnis von 0,1 bis 20 Molprozent bezogen auf die Verbindung der allgemeinen Formel (III) eingesetzt. Bevorzugt sind 0,1 bis 10 Molprozent, besonders bevorzugt 5 bis 10 Molprozent.

Die Reaktion wird in der Regel bei einer Temperatur von -60 bis -80° C durchgeführt. Erfolgt eine Ummetallierung auf M² = Zn oder Mg kann die Verbindung der allgemeinen Formel (V) auch auf 0 bis 23 °C erwärmt werden ohne dass verstärkt Zersetzung eintritt.

Besonders bevorzugt ist folgende Kombination aus den Gruppen der oben beschriebenen Metallierungsreagenzien, Lösungsmittel, Amine sowie Elektrophile: n-Butyllithium, THF in Kombination mit einem C₆-C₈ Kohlenwasserstoff, Diisopropylamin oder 2,2,6,6-Tetramethylpiperidin sowie Kohlendioxid.

### Dritter Schritt des erfindungsgemäßen Verfahrens:

Die Verbindung der Formel (VI) wird mit einem Acyltransfer Reagenz der allgemeinen Formel (VIII), einer Base und einem 5-Amino-2-alkyl-1H-tetrazol der allgemeinen Formel (VII) in einem geeigneten Lösungsmittel vorgelegt. Dann wird ein aktivierendes Reagens (Aktivator) langsam zudosiert und, gegebenenfalls bei erhöhter Temperatur, weiter gerührt.

Die Verbindungen der Formeln (VI) und (VII) werden üblicherweise in einem Molverhältnis von 0,8 bis 1,5 eingesetzt. Vorzugsweise werden sie äquimolar eingesetzt.

Als Acyltransfer Reagens können N1-substituierte Imidazole der allgemeinen Formel (VIII) verwendet werden.

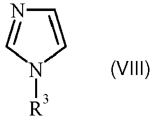

Darin steht R³ für C₁-C₁₂-Alkyl oder Phenyl. Bevorzugt steht R³ für Methyl. Alternativ dazu kann beispielsweise 4-N,N-dimethylaminopyridin verwendet werden. Das Acyltransfer Reagens der Formel (VIII) und die Verbindung der Formel (VI) werden üblicherweise in einem Molverhältnis von 0,5 bis 10, bevorzugt von 1 bis 3, besonders bevorzugt von 1 bis 2 eingesetzt. Bei der Verwendung von Tributylamin als Base ist ein Molverhältnis von 1,0 besonders bevorzugt. Bei der Verwendung von 3-Picolin als Base ist ein Molverhältnis von 2,0 besonders bevorzugt.

Als Base sind aromatische Amine wie Pyridin oder Picoline sowie tertiäre Amine wie Triethylamin, Tributylamin oder Diisopropylethylamin geeignet. Besonders geeignet sind 3-Picolin oder Tributylamin. Die Base wird in einem Mengenverhältnis von 2:1 bis 4:1 Moläquivalenten bezogen auf die Verbindung (VI) eingesetzt. Bevorzugt ist ein Mengenverhältnis von 2:1 bis 3:1. Bei der Verwendung von 3-Picolin als Base ist ein Mengenverhältnis von 2,5:1 besonders bevorzugt. Bei der Verwendung von Tributylamin als Base ist ein Mengenverhältnis von 3:1 besonders bevorzugt. Wird das Acyltransfer Reagens der Formel (VIII) und die Verbindung der Formel (VI) in einem Molverhältnis ≥ 4,5 eingesetzt, ist keine Zugabe einer Base erforderlich.

Als Aktivator geeignet sind Thionylchlorid, Phosgen, Diphosgen, Mesylchlorid, POCl₃, PCl₅ und Oxalylchlorid. Bevorzugt verwendet werdenThionylchlorid oder Phosgen. Besonders bevorzugt wird Thionylchlorid verwendet. Der Aktivator wird in einem Mengenverhältnis von 0,5:1 bis 3:1 Moläquivalenten bezogen auf die Verbindung (VI) eingesetzt. Bevorzugt ist ein Mengenverhältnis von 1:1 bis 2:1, besonders bevorzugt 1,2:1 bis 1,9:1.

Als Lösungsmittel geeignet sind inerte organische Lösungsmittel, vorzugsweise aliphatische, alicyclische oder aromatische Kohlenwasserstoffe, wie Petrolether, Hexan, Heptan, Cyclohexan, Methylcyclohexan, Benzol, Toluol, Xylol und Decalin; halogenierte Kohlenwasserstoffe, wie Chlorbenzol, Dichlorbenzol, Dichlormethan, Chloroform, Tetrachlormethan, 1,2-Dichlorethan und Trichlorethan; Ether wie Diethylether, Diisopropylether, Methyl-t-butylether, Methyl-t-amylether, Dioxan, THF, Methyl-THF, 1,2-Dimethoxyethan, 1,2-Diethoxyethan und Anisol; Ketone wie Aceton, und Methyl-isobutylketon; Nitrile wie Acetonitril, Propionitril, n- oder i-Butyronitril und Benzonitril; Amide wie N,N-Dimethylformamid, N,N-Dimethylacetamid, N-Methylformanilid, N-Methylpyrrolidin und Hexamethylphosphoramid; Pyridine wie 2-Methylpyridin, 3-Methylpyridin, 4-Methylpyridin, 2,3-Dimethylpyridin, 2-Methyl-5-ethylpyridin, 2,6-Dimethylpyridin, 2,4-Dimethylpyridin, 3,4-Dimethylpyridin und 2,4,6-Trimethylpyridin. Vorzugsweise wird als Lösungsmittel THF, Acetonitril oder 3-Methylpyridin verwendet. Besonders bevorzugt wird Acetonitril verwendet.

Die Reaktion wird üblicherweise bei einer Temperatur von -5 ° bis 80 °C durchgeführt. Bei der Verwendung von 3-Methylpyridin als Lösungsmittel wird die Reaktion vorzugsweise bei 0 bis 25 °C durchgeführt und die vollständige Umsetzung ist in der Regel nach 10 bis 20 Stunden erfolgt. Bei der Verwendung von Acetonitril als Lösungsmittel und Tributylamin als Base wird die Reaktion vorzugsweise bei 0 bis 25 °C durchgeführt und die vollständige Umsetzung ist in der Regel nach 1 bis 5 Stunden erfolgt. Bei der Verwendung von Acetonitril als Lösungsmittel und 3-Methylpyridin als Base wird die Reaktion vorzugsweise bei 60 bis 80 °C durchgeführt und die vollständige Umsetzung ist in der Regel nach 4 bis 8 Stunden erfolgt.

Die Aufarbeitung der Reaktion erfolgt nach einem in den Beispielen beschriebenen Verfahren.

Verbindungen der Formel (III) sind neu und eignen sich sehr gut als Ausgangsmaterial für den zweiten Schritt des erfindungsgemäßen Verfahrens. Ein weiterer Gegenstand vorliegender Erfindung sind somit Verbindungen der Formel (III) 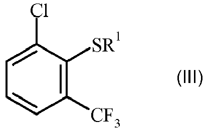 worin
R¹ bedeutet C₁-C₄-Alkyl oder durch s Reste aus der Gruppe bestehend aus Chlor, Fluor, Methoxy und Ethoxy substituiertes Phenyl,
s bedeutet 0, 1, 2 oder 3.

Bevorzugt steht R¹ für C₁-C₄-Alkyl. Besonders bevorzugt steht R¹ für Methyl.

Verbindungen der Formel (VI) sind ebenfalls neu und eignen sich sehr gut als Ausgangsmaterial für den dritten Schritt des erfindungsgemäßen Verfahrens. Ein weiterer Gegenstand vorliegender Erfindung sind somit Verbindungen der Formel (VI) 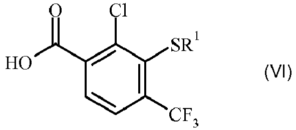 worin
R¹ bedeutet C₁-C₄-Alkyl oder durch s Reste aus der Gruppe bestehend aus Chlor, Fluor, Methoxy und Ethoxy substituiertes Phenyl,
s bedeutet 0, 1, 2 oder 3.

Bevorzugt steht R¹ für C₁-C₄-Alkyl. Besonders bevorzugt steht R¹ für Methyl.

Die nachfolgenden Beispiele erläutern die Erfindung näher ohne sie darauf einzuschränken.

### Herstellung von 2-Chlor-3-(methylsulfanyl)-N-(1-methyl-1H-tetrazol-5-yl)-4-(trifluormethyl)benzamid

### Schritt 1: 3-Chlor-2-(methylsulfanyl)-benzotrifluorid

500.0 g (2.28 mol, 1.0 eq) 2,3-Dichlortrifluor-methylbenzol und 50.0 g (0.06 mol, 2.5 mol%) wässrige 50% CYPHOS® (Tetradecyl-tri-n-butylphosphonium chlorid) Lösung werden unter Stickstoff vorgelegt und auf 80 °C erhitzt. 990.0 g (2.96 mol, 1.3 eq) wässrige 21% Natriumthiomethylat Lösung wird bei 80 °C innerhalb von 2h zudosiert und weitere 4h bei 80 °C gerührt. Die organische Phase wird abgelassen und die wässrige Phase mit 300 ml Toluol extrahiert. Die vereinigten organischen Phasen werden vereint und bei 40 °C / 50 mbar eingeengt. Der Rückstand wird im Vakuum bei 10 mbar destilliert. Man erhält 361 g einer farblosen Flüssigkeit (Sdp. 104 °C / 10 mbar) in einer Ausbeute von 70%.
¹H-NMR (CDCl3, 400 MHz) δ (ppm) = 7.67 (dd, *J* = 8.1, 1.3 Hz, 1H), 7.64 (dd, *J* = 8.0, 1.3 Hz, 1H), 7.38 (td, *J* = 8.0, 0.8 Hz, 1H), 2.42 (s, 3H).

### Schritt 2: 2-Chlor-4-trifluormethyl-3-methylsulfanyl-benzoesäure

Eine Lösung von 100.0 g (0.44 mol, 1.0 eq) 1-chlor-2-(methylsulfanyl)-3-(trifluoromethyl)benzol und 4.5 g (0.04 mol, 0.1 eq) Diisopropylamin werden in 500 ml THF unter Stickstoff vorgelegt und auf -70 °C abgekühlt. 122.3 g (0.44 mol, 1.0 eq) 23% n-Butyllithium Lösung in Hexan wird bei -70 °C über einen Zeitraum von 3h zudosiert und im Anschluß 2h bei -70 °C nachgerührt. Man erhält eine orangerötliche Suspension. CO₂ Gas wird anschließend oberhalb der Reaktionslösung so in den Kolben eingeleitet, dass die Temperatur -60 °C nicht überschreitet. Nach ca. 1h ist keine Exothermie mehr erkennbar und das Reaktionsgemisch wird innerhalb von 1h auf 23 °C erwärmt. Man erhält eine cremefarbene Suspension. 500 ml Methylcyclohexan werden zugegeben und dann das Gemisch eingeengt beginnend bei 40 °C / 400 mbar bis auf 40 °C / 150 mbar. Die blassgelbe Suspension wird mit 500 ml Wasser versetzt und 10 min bei 23 °C gerührt, wobei der Feststoff in Lösung geht. Die organische Phase wird abgetrennt und verworfen. 100 ml 20% HCl werden innerhalb von 1h zur wässrigen Phase zudosiert (pH = 1- 2). Der farblose Feststoff wird abfiltriert, mit 350 ml 40 °C warmen Wasser gewaschen und bei 10 mbar / 40 °C getrocknet. Man erhält 99.3 g Produkt (81% Ausbeute).
¹H-NMR (DMSO-d₆, 400 MHz) δ (ppm) = 14.02 (s br, 1H), 7.88 (d, J = 8.3 Hz, 1H), 7.85 (d, J = 8.1 Hz, 1H), 2.42 (s, 3H).

### Schritt 3: 2-Chlor-3-(methylsulfanyl)-N-(1-methyl-1H-tetrazol-5-yl)-4-(trifluormethyl)benzamid

Eine Lösung von 100.0 g 2-Chlor-3-(methylsulfanyl)-4-(trifluormethyl)benzoesäure (0,362 mol, 1.0 eq), 84.3 g 3-Picolin (0.905 mol, 2.5 eq), 59.5 g 1-Methyl-1*H*-imidazol (0.724 mol, 2.0 eq) und 41.5 g 5-Amino-1-methyl-1*H*-tetrazol (95%, 0.398 mol, 1.1 eq) werden in 640 ml Acetonitril unter Stickstoff vorgelegt und auf Rückfluss erhitzt. 68.9 g Thionylchlorid (0.579 mol, 1.6 eq) wird innerhalb von 3h zudosiert. Anschließend wird 3h bei 74 °C gerührt. Dann wird bei 40 °C / 50 mbar ca. 80 - 90% Acetonitril abdestilliert bis auf ein Restgewicht von ca. 350 - 400 g. 400 ml 10% HCl werden bei 23 °C innerhalb von 5h zudosiert und 1h bei 23 °C nachgerührt. Der beige-farbene Feststoff wird abfiltriert, nacheinander mit 500 ml 10% HCl sowie 400 ml Wasser gewaschen und anschließend bei 40 °C / 10 mbar getrocknet. Man erhält 124 g der stabilen Kristallmodifikation in einer Ausbeute von 95%.
¹H-NMR (CDCl₃, 400 MHz) δ (ppm) = 11.25 (s br, 1H), 7.80 (d, J = 8.3 Hz, 1H), 7.73 (d, J = 8.1 Hz, 1H), 4.15 (s, 3H), 2.45 (s, 3H).

## Patentansprüche

1. Verfahren zur Herstellung von 3-Alkylsulfanyl-2-chlor-N-(1-alkyl-1*H*-tetrazol-5-yl)-4-trifluoromethyl-benzamiden der allgemeinen Formel (I), **dadurch gekennzeichnet, dass**
a) in einem ersten Schritt 2,3-Dichlorbenzotrifluorid (II) mit einem Thiolat (IV) zu einem 2-Alkysulfanyl-3-chlor-benzotrifluorid (III) umgesetzt wird,
b) in einem zweiten Schritt das 2-Alkysulfanyl-3-chlor-benzotrifluorid (III) mit einem metallorganischen Reagens zu [2-Chlor-3-(alkylsulfanyl)-4-(trifluormethyl)phenyl] Metall Anion (V) und anschließend mit einem Carboxlierungsreagenz zu 3-Alkylsulfanyl-2-chlor-4-trifluormethylbenzoesäure (VI) umgesetzt wird, und
c) in einem dritten Schritt mit einem Aktivator in Anwesenheit einer Base und eines Acyltransfer Reagenz die Amidierung mit einem 5-Amino-1-alkyltetrazol (VII) zum 3-Alkylsulfanyl- 2-chlor-N-(1-alkyl-1*H*-tetrazol-5-yl)-4-trifluoromethyl-benzamid (I) erfolgt: 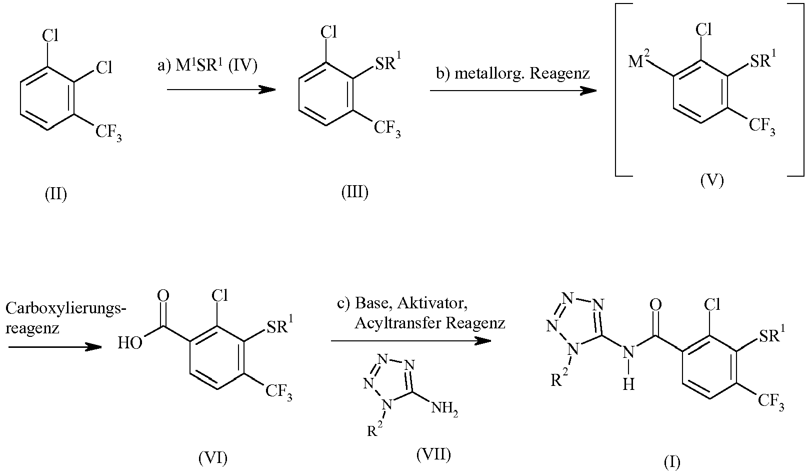 und
d) worin die Substituenten wie nachfolgend definiert sind:
R¹ bedeutet C₁-C₄-Alkyl oder durch s Reste aus der Gruppe bestehend aus Chlor, Fluor, Methoxy und Ethoxy substituiertes Phenyl,
R² bedeutet C₁-C₄-Alkyl oder durch s Reste aus der Gruppe bestehend aus Chlor, Fluor, Methoxy und Ethoxy substituiertes Phenyl,
M¹ bedeutet Lithium, Natrium oder Kalium,
M² bedeutet Li, ZnX, MgX,
X bedeutet Chlor, Brom oder Iod,
s bedeutet 0, 1, 2 oder 3.

2. Verfahren nach Anspruch 1, worin als Thiolat (IV) NaSMe oder KSMe verwendet wird.

3. Verfahren nach Anspruch 1 oder 2, worin R¹ für C₁-C₄-Alkyl steht.

4. Verfahren nach einem der Ansprüche 1 bis 3, worin R¹ für Methyl steht.

5. Verfahren nach einem der Ansprüche 1 bis 4, worin M¹ für Natrium steht.

6. Verfahren nach einem der Ansprüche 1 bis 5, worin R² für Methyl steht.

7. Verfahren nach einem der Ansprüche 1 bis 6, worin eine Alkyllithium Verbindung als metallorganisches Reagenz verwendet wird.

8. Verfahren nach einem der Ansprüche 1 bis 7, worin Kohlendioxid als Carboxylierungsreagenz verwendet wird.

9. Verfahren nach einem der Ansprüche 1 bis 8, worin Thionylchhlorid als Aktivator verwendet wird.

10. Verfahren nach einem der Ansprüche 1 bis 9, worin 1-Methyl-1*H-*imidazol als Acyltransfer Reagenz verwendet wird.

11. Verbindungen der Formel (III), 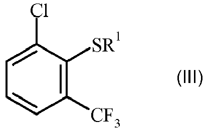 worin
R¹ bedeutet C₁-C₄-Alkyl oder durch s Reste aus der Gruppe bestehend aus Chlor, Fluor, Methoxy und Ethoxy substituiertes Phenyl,
s bedeutet 0, 1, 2 oder 3.

12. Verbindungen der Formel (III) nach Anspruch 11, worin R¹ für Methyl steht.

13. Verbindungen der Formel (VI) 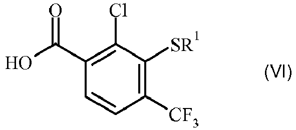 worin
R¹ bedeutet C₁-C₄-Alkyl oder durch s Reste aus der Gruppe bestehend aus Chlor, Fluor, Methoxy und Ethoxy substituiertes Phenyl,
s bedeutet 0, 1, 2 oder 3.

14. Verbindungen der Formel (VI) nach Anspruch 13, worin R¹ für Methyl steht.

## Claims

1. Method for preparing 3-alkylsulphanyl-2-chloro-N-(1-alkyl-1*H*-tetrazol-5-yl)-4-trifluoromethylbenzamides of the general formula (I), **characterized in that**
a) in a first step 2,3-dichlorobenzotrifluoride (II) is reacted with a thiolate (IV) to give a 2-alkylsulphanyl-3-chlorobenzotrifluoride (III),
b) in a second step the 2-alkylsulphanyl-3-chlorobenzotrifluoride (III) is reacted with an organometallic reagent to give [2-chloro-3-(alkylsulphanyl)-4-(trifluoromethyl)phenyl] metal anion (V) and subsequently with a carboxylating reagent to give 3-alkylsulphanyl-2-chloro-4-trifluoromethylbenzoic acid (VI), and
c) in a third step amidation with a 5-amino-1-alkyltetrazole (VII) is effected with an activator in the presence of a base and an acyl transfer reagent to give 3-alkylsulphanyl- 2-chloro-N-(1-alkyl-1*H*-tetrazol-5-yl)-4-trifluoromethylbenzamide (I) : 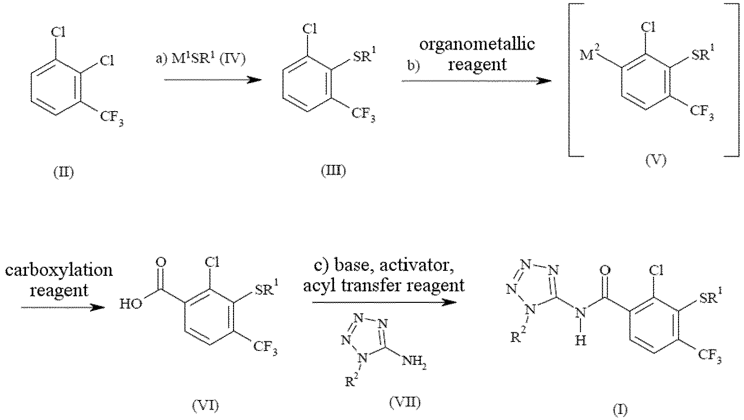 and
d) where the substituents are as defined hereinbelow:
R¹ is C₁-C₄-alkyl or phenyl substituted by s radicals from the group consisting of chlorine, fluorine, methoxy and ethoxy,
R² is C₁-C₄-alkyl or phenyl substituted by s radicals from the group consisting of chlorine, fluorine, methoxy and ethoxy,
M¹ is lithium, sodium or potassium,
M² is Li, ZnX, MgX,
X is chlorine, bromine or iodine,
s is 0, 1, 2 or 3.

2. Method according to Claim 1, wherein the thiolate (IV) used is NaSMe or KSMe.

3. Method according to Claim 1 or 2, where R¹ is C₁-C₄-alkyl.

4. Method according to any of Claims 1 to 3, wherein R¹ is methyl.

5. Method according to any of Claims 1 to 4, wherein M¹ is sodium.

6. Method according to any of Claims 1 to 5, wherein R² is methyl.

7. Method according to any of Claims 1 to 6, wherein the organometallic reagent used is an alkyllithium compound.

8. Method according to any of Claims 1 to 7, wherein the carboxylating reagent used is carbon dioxide.

9. Method according to any of Claims 1 to 8, wherein the activator used is thionyl chloride.

10. Method according to any of Claims 1 to 9, wherein the acyl transfer reagent used is 1-methyl-1*H*-imidazole.

11. Compounds of the formula (III), 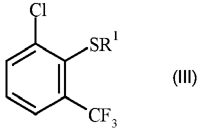 in which
R¹ is C₁-C₄-alkyl or phenyl substituted by s radicals from the group consisting of chlorine, fluorine, methoxy and ethoxy,
s is 0, 1, 2 or 3.

12. Compounds of the formula (III) according to Claim 11, where R¹ is methyl.

13. Compounds of the formula (VI) 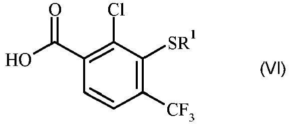 in which
R¹ is C₁-C₄-alkyl or phenyl substituted by s radicals from the group consisting of chlorine, fluorine, methoxy and ethoxy,
s is 0, 1, 2 or 3.

14. Compounds of the formula (VI) according to Claim 13, where R¹ is methyl.

## Revendications

1. Procédé pour la préparation de 3-alkylsulfanyl-2-chloro-N-(1-alkyl-1H-tétrazol-5-yl)-4-trifluorométhylbenzamides de formule générale (I), **caractérisé en ce que**
a) dans une première étape le 2,3-dichlorobenzotrifluorure (II) est transformé avec un thiolate (IV) pour donner un 2-alkylsulfanyl-3-chlorobenzotrifluorure (III),
b) dans une deuxième étape le 2-alkylsulfanyl-3-chlorobenzotrifluorure (III) est transformé avec un réactif organométallique en anion métallique (V) de [2-chloro-3-(alkylsulfanyl)-4-(trifluorométhyl)phényle] et ensuite avec un réactif de carboxylation en acide 3-alkylsulfanyle-2-chloro-4-trifluorométhylbenzoïque (VI), et
c) dans une troisième étape avec un activateur en présence d'une base et d'un réactif de transfert d'acyle, l'amidation avec un 5-amino-1-alkyltétrazole (VII) en 3-alkylsulfanyl-2-chloro-N-(1-alkyl-1H-tétrazol-5-yl)-4-trifluorométhylbenzamide (I) est réalisée : 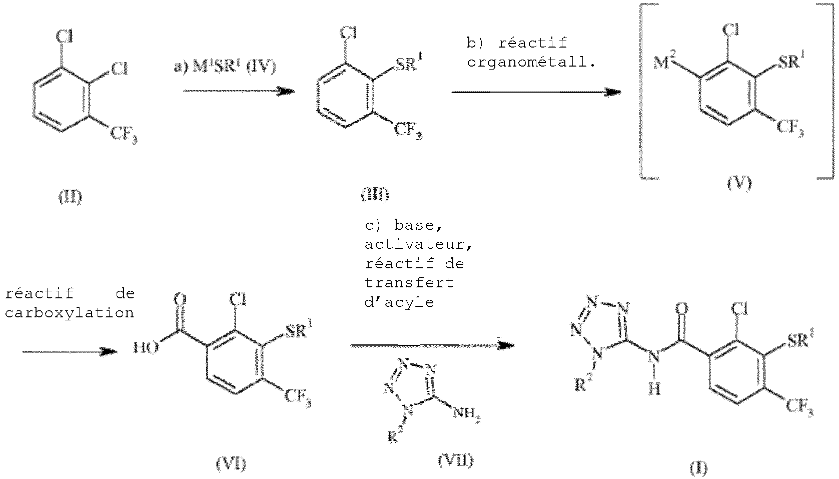 et
d) dans lequel les substituants sont définis comme suit :
R¹ signifie C₁-C₄-alkyle ou phényle substitué par s radicaux du groupe constitué par chlore, fluor, méthoxy et éthoxy,
R² signifie C₁-C₄-alkyle ou phényle substitué par s radicaux du groupe constitué par chlore, fluor, méthoxy et éthoxy,
M¹ signifie lithium, sodium ou potassium,
M² signifie Li, ZnX, MgX,
X signifie chlore, brome ou iode,
s signifie 0, 1, 2 ou 3.

2. Procédé selon la revendication 1, NaSMe ou KSMe étant utilisé en tant que thiolate (IV).

3. Procédé selon la revendication 1 ou 2, R¹ représentant C₁-C₄-alkyle.

4. Procédé selon l'une quelconque des revendications 1 à 3, R¹ représentant méthyle.

5. Procédé selon l'une quelconque des revendications 1 à 4, M¹ représentant sodium.

6. Procédé selon l'une quelconque des revendications 1 à 5, R² représentant méthyle.

7. Procédé selon l'une quelconque des revendications 1 à 6, un composé de type alkyllithium étant utilisé en tant que réactif organométallique.

8. Procédé selon l'une quelconque des revendications 1 à 7, du dioxyde de carbone étant utilisé en tant que réactif de carboxylation.

9. Procédé selon l'une quelconque des revendications 1 à 8, du chlorure de thionyle étant utilisé en tant qu'activateur.

10. Procédé selon l'une quelconque des revendications 1 à 9, le 1-méthyl-1*H*-imidazole étant utilisé en tant que réactif de transfert d'acyle.

11. Composés de formule (III), 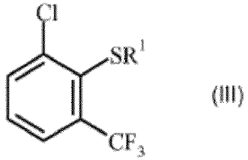 dans laquelle
R¹ signifie C₁-C₄-alkyle ou phényle substitué par s radicaux du groupe constitué par chlore, fluor, méthoxy et éthoxy,
s signifie 0, 1, 2 ou 3.

12. Composés de formule (III) selon la revendication 11, dans laquelle R¹ représente méthyle.

13. Composés de formule (VI) 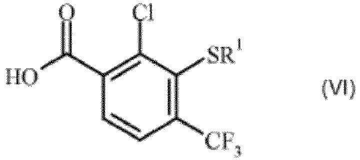 dans laquelle
R¹ signifie C₁-C₄-alkyle ou phényle substitué par s radicaux du groupe constitué par chlore, fluor, méthoxy et éthoxy,
s signifie 0, 1, 2 ou 3.

14. Composés de formule (VI) selon la revendication 13, dans laquelle R¹ représente méthyle.
